# EUROPEAN PATENT APPLICATION

(11) **EP 2 320 230 A1**
(43) Date of publication of application: **11.05.2011**
(21) Application number: 09802921.8
(22) Date of filing: 27.07.2009
(51) Int. Cl.: G01N 33/50, C12Q 1/04, C12Q 1/68, G01N 33/48, G01N 33/483

(54) **REAGENT FOR DETECTING ABNORMAL CELL IN CERVIX OF UTERUS, AND METHOD FOR DETECTING ABNORMAL CELL IN CERVIX OF UTERUS BY USING SAME**

(30) Priority: 30.07.2008 JP 2008196372
(71) Applicant: Sysmex Corporation, Kobe-shi, Hyogo 651-0073 (JP)
(72) Inventor: MORITA, Masakatsu, Kobe-shi Hyogo 651-0073 (JP); KAWAI, Akinori, Kobe-shi Hyogo 651-0073 (JP); TSUJINO, Yukio, Kobe-shi Hyogo 651-0073 (JP)
(74) Representative: Paemen, Liesbet R.J.
(86) International application number: PCT/JP2009/063353
(87) International publication number: WO 2010/013678

(57) **Abstract**

Disclosed is a reagent for detecting an abnormal cell in the cervix of uterus, which can detect an abnormal cell contained in a biological sample that contains cells collected from the cervix of uterus. The reagent comprises a dye represented by general formula (I).

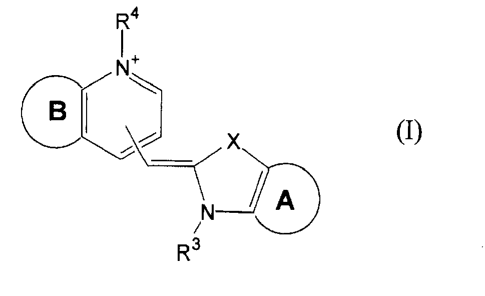

## Description

### TECHNICAL FIELD

The present invention relates to a reagent for detecting abnormal cells such as cancer cells or atypical cells in a biological sample obtained from the uterine cervix, and to a method for detecting abnormal cells in a sample obtained from the uterine cervix using the same.

### BACKGROUND ART

Cervical cancer is a cancer developed in the cervical surface epithelium and most of the cases are squamous cell cancer. In the conventional examinations for cervical cancer, cells taken from the uterine cervix are subjected to the screening by cytological diagnosis. When the cells are diagnosed to be abnormal, then detailed examinations such as histological diagnosis are carried out.

In the histological diagnosis of the uterine cervix, the cells are classified into three stages, i.e. mild, moderate and severe dysplasias, as the premalignant stages, according to the extent of the emergence of atypical cells in epithelium. When the pathology is exacerbated beyond severe dysplasia, it comes to the stage in which cancer cells appear in epithelium. The pathology proceeds to "intraepithelial cancer" in which cancer cells are localized in epithelium and then to "invasive cancer (so-called cervical cancer)" in which cancer cells invade from epithelium to the subcutaneous tissue.

The cytological diagnosis which is carried out as a screening examination in order to decide whether or not such histological diagnosis is carried out is carried out by smearing a biological sample from the uterine cervix to a slide glass and examining the same under a microscope.
The cytological diagnosis requires the decision by an examiner based on his/her observation, and has possibilities that cervical cancer is overlooked due to, for instance, the preparation of the examination samples. In addition, because it requires skill of examiners, it is difficult to carry out rapid and highly precise examinations of many samples. Further, it is highly possible that such skilled examiners may be shorthanded.

Accordingly, attempts have been made in order to detect abnormal cells in biological samples from the uterine cervix by automated systems as a support for cytological diagnosis.
In the known methods of detecting abnormal cells which may be used in automated systems, DNA in the nuclei of cells is stained with a fluorescent dye, the amount of DNA is calculated based on the fluorescence measured with flow cytometry and cancer cells are detected based on the calculated DNA amount.

W. A. Linden et al. (Non Patent Literature 1) detected cancer cells by measuring fluorescence of DNA of the cells which are obtained from the uterine cervix and stained with ethidium bromide, and also detected cancer cells based on the amounts of DNA and protein which have been simultaneously stained with ethidium bromide and fluorescein isothiocyanate (FITC), respectively.
B. J. Fowlkes et al. (Non Patent Literature 2) detected cancer cells based on two kinds of fluorescent intensities measured by a flow cytometer of the nuclei and cytoplasm of the cells which have been stained with propidium iodide (PI) and FITC, respectively.

However, these methods have not been successfully applied to the automated cervical cancer detection system because of high rates of false positives and false negatives.

U.S. Patent No. 4,883,867 (Patent Literature 1) discloses that reticulocytes are stained and detected with the dye of the following general formula: 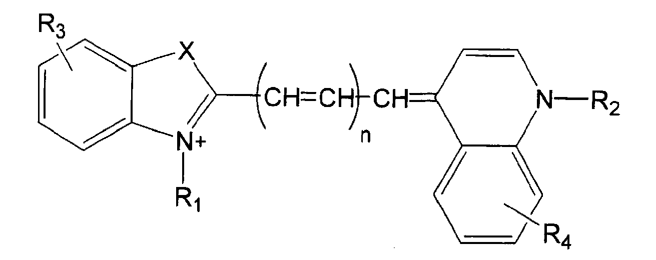 wherein:
X is O, S, Se, N-alkyl (having 1-6 carbons) or C(CH₃)ₙ; R₁ is C₁₋₆ alkyl;
R₂ is C₁₋₆ alkyl; R₃ is a fused benzene, C₁₋₆ alkyl or methoxy or is absent; R₄ is C₁₋₆ alkyl or methoxy or is absent; and n is 0 or an integer of 1 to 6.

### CITATION LIST

### Patent Literature

Patent Literature 1: U.S. Patent No. 4,883,867

### Non Patent Literature

Non Patent Literature 1: W. A. Linden et al., J. Histochem. Cytochem., 1979, Vol. 27, p.529-535
Non Patent Literature 2: B. J. Fowlkes et al., J. Histochem. Cytochem., 1976, Vol. 24, p. 322-331

### SUMMARY OF INVENTION

### Technical Problem

The present invention is to provide a reagent which allows more sensitive detection of abnormal cells in biological samples obtained from the uterine cervix and a method of detecting cervical abnormal cells using such reagent.

### Solution to Problem

In order to solve the above problem, the present inventors focused their attention on the following point.
Thus, it is generally known that the cancerous changes in the cells lead to a defect in their nuclei (e.g. change in the size of nuclei, increase in DNA amount, chromatin condensation etc.). Then, the inventors considered that cancer cells may be distinguished from normal cells based on these phenomena.
They found that such abnormalities in nuclei can be detected by using a compound represented by the following general formula (I):

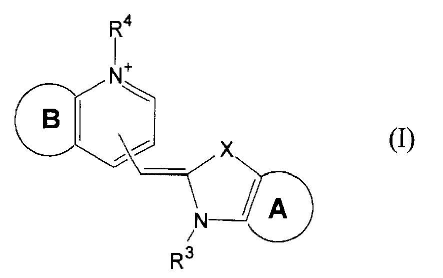

wherein:
X represents S or O;

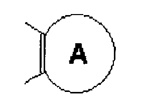

represents 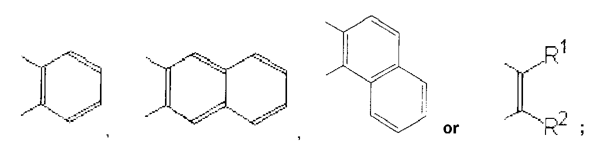 R¹ and R², which are identical or different, represent a hydrogen atom or a C₁₋₆ alkyl group;

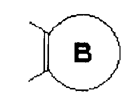

represents 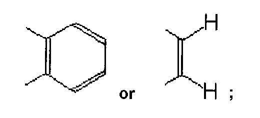 and
R³ and R⁴, which are identical or different, represent a linear or branched C₁₋₆ alkyl group which may have a sulphonic acid group as a substituent,
or a salt thereof as a nucleic acid staining dye, allowing the detection of cervical abnormal cells with higher sensitivity, and completed the present invention.

Thus, the present invention provides a cervical abnormal cell detecting reagent for detecting abnormal cells in a biological sample containing cells from the uterine cervix, which comprises a dye having the above general formula (I).
The present invention also provides a method of detecting abnormal cells in a biological sample containing cells obtained from the uterine cervix comprising the steps of:
preparing a measurement sample by bringing the biological sample into contact with the above cervical abnormal cell detecting reagent;
allowing the measurement sample to flow through a flow cell, irradiating light to the measurement sample in the flow cell and detecting fluorescence emitted from the measurement sample;
obtaining information relating to cell nuclei based on a fluorescent signal generated from the fluorescence detected; and
detecting the abnormal cells in the biological sample based on the obtained information relating to nuclei.

### Advantageous Effects of Invention

By using the cervical abnormal cell detecting reagent of the present invention, abnormal cells in biological samples obtained from the uterine cervix can be detected with higher sensitivity. The method of detecting abnormal cells using the present reagent can be used for more accurate diagnoses for cervical cancer due to the increased sensitivity for the detection of abnormal cells. The present method can also be automated, allowing rapid and convenient detection of cervical cancer.

### BRIEF DESCRIPTION OF DRAWINGS

Fig. 1 shows the rate of abnormal cells obtained by using the present cervical abnormal cell detecting reagents (Examples 1 and 2) and a comparative reagent (Comparative Example 1).
Fig. 2 shows the rate of abnormal cells obtained by using the present cervical abnormal cell detecting reagent (Example 3).
Fig. 3 shows the rate of abnormal cells obtained by using the present cervical abnormal cell detecting reagent (Example 4).
Fig. 4 shows an example of a fluorescent signal waveform to be detected in a flow cytometer.
Fig. 5 shows fluorescent intensities of DNAs stained with propidium iodide (PI) and PI + Dye A.
Fig. 6 shows results of ROC analysis of the judgments based on (1) the fluorescence peak area values (Δ), (2) the fluorescence peak area values and fluorescence peak values (0) and (3) the fluorescence peak area values, fluorescence peak values and the fluorescence pulse widths (•).

### DESCRIPTION OF EMBODIMENTS

The abnormal cells which can be detected with the present cervical abnormal cell detecting reagent (hereinafter simply referred to as "reagent") are pathological epithelial cells which are considered to reflect defects in the uterine cervix, and include cancer cells and atypical cells. As used herein, the term "atypical cells" has the meaning known in the art, and they are pathological epithelial cells which are, although they are not cancer cells, considered to develop into cancer cells. These abnormal cells are known to have abnormalities in their nuclei (e.g. change in the size of nuclei, increase in DNA amount, chromatin condensation etc.).

The present reagent comprises the dye having the above general formula (I).
In the general formula (I), X is an oxygen or sulphur atom.
In the general formula (I), R¹ and R², which are identical or different, represent a hydrogen atom or a C₁₋₆ alkyl group.
In the general formula (I), R³ and R⁴, which are identical or different, represent a C₁₋₆ alkyl group which may have a sulphonic acid group as a substituent.

As used herein, "C₁₋₆ alkyl group" means a linear or branched alkyl group having 1 to 6 carbon atoms. Such alkyl group includes methyl, ethyl, propyl, isopropyl, butyl, isobutyl, sec-butyl, tert-butyl, pentyl, isopentyl, tert-pentyl, hexyl, isohexyl and the like.
As used herein, "sulphonic acid group" means -SO₃H or -SO₃-.

In the above general formula (I), the structure containing a thiazolyl or oxazolyl group with the A group may be in ortho-, meta- or para-position, more preferably in ortho- or para-position to the nitrogen atom of the pyridine ring having the B group.

The compounds represented by the general formula (I) may be in the form of a salt. In such case, the counter ion may be either of anions such as halogen ions, a tosylate anion and cations such as a triethylammonium ion.
More preferably, the compound represented by the general formula (I) is a salt and the counter ion is an anion. As the anion, an iodine ion is preferred.

The compounds of the general formula (I) or salts thereof are considered to have high specificity towards DNAs due to its binding ability to a minor groove of double helix of DNAs. Due to such nature, it is considered that the compounds contained in the present reagent can specifically stain DNAs.

The preferable examples of the compounds of the formula (I) are the followings:
Dye A (CAS No. 15941-82-9) represented by the following formula:

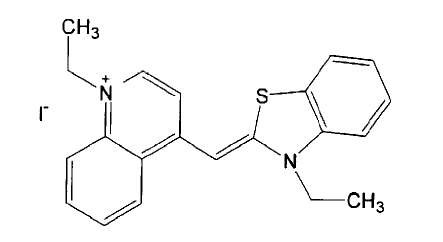

Dye B (CAS No. 24147-36-2; thiazole orange):

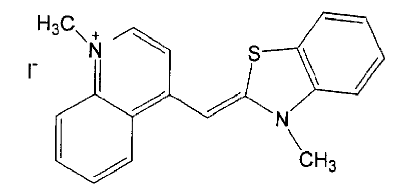

Dye C (CAS No. 143413-86-9; oxazole yellow):

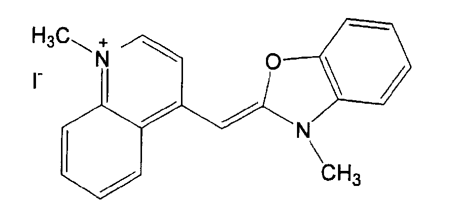

Dye D (CAS No. 223654-13-5):

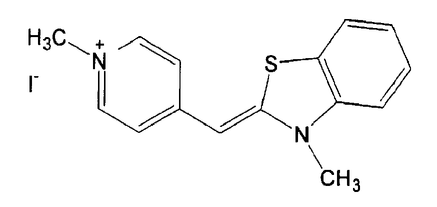

Dye E (CAS No. 97214-99-8):

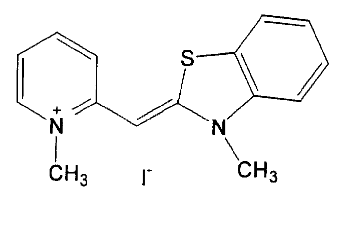

Dye F (CAS No. 16768-72-2):

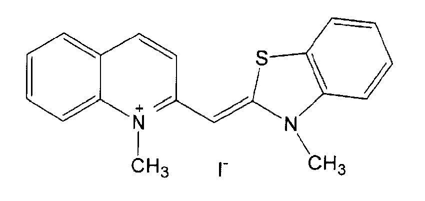

Dye G:

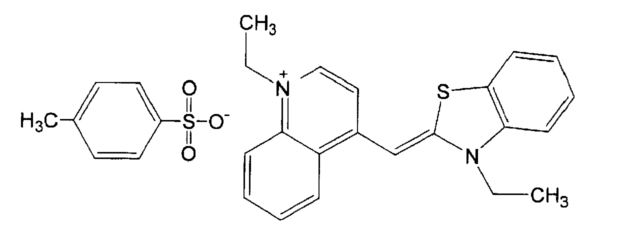

Dye H:

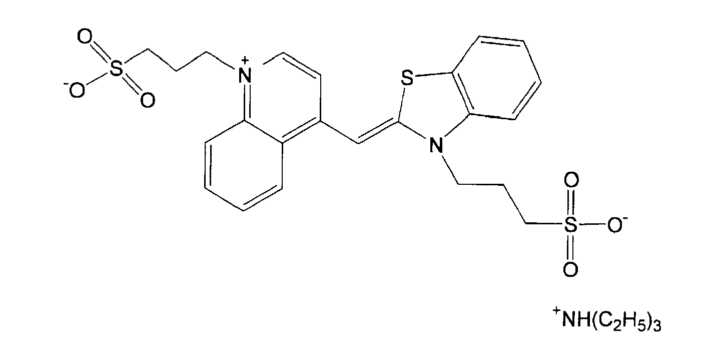

Dye J:

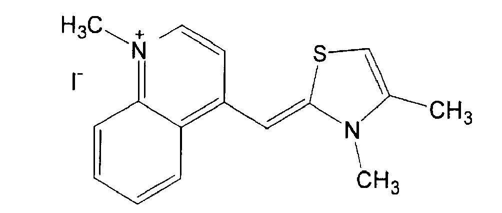

Dye K:

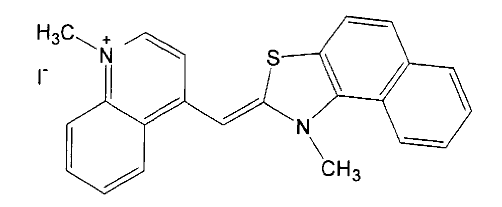

Dye L:

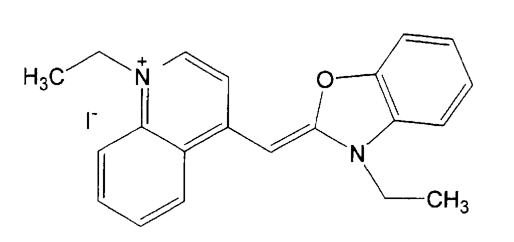

The above compounds represented by the formula (I) or salts thereof may be produced according to the conventional synthetic methods in organic chemistry. The above specific Dyes A to L can be purchased from Hayashibara Biochemical Laboratories, Inc. Specific production examples of Dyes A to L are shown in Examples.

It is preferable that the present reagent further comprises, in addition to the dye of the general formula (I), an intercalating dye. As used herein, "intercalating dye" means a dye which can intercalate into the hydrogen bond of a DNA base pair to form a bond with the base. The intercalating dye is preferably a dye selected from propidium iodide (PI), ethidium bromide and acridine orange, and among which PI is more preferable.

The concentration of the dye of the formula (I) and the optional intercalating dye in the present reagent is preferably such concentration that the total concentration of 0.01 to 100 mg/ml, more preferably 0.01 to 10 mg/ml, and still more preferably 0.1 to 10 mg/ml is achieved when the reagent is mixed with a biological sample containing cells from the uterine cervix.

The mixing ratio (weight ratio) of the dye of the formula (I) and the optional intercalating dye is preferably the dye of the formula (I) : intercalating dye = 1 : 0 to 100, and more preferably 1 : 10 to 30.

The present reagent preferably has pH of 6 to 9, and more preferably 7 to 8.5. When the reagent has such pH, the morphology of cell nuclei can be maintained, so that the fluorescence from the cell nuclei stained with the reagent can be stabilized.

In order to adjust pH of the reagent within the above range, the present reagent may comprise an appropriate buffering agent. The buffering agent may be the one which has a buffering capacity at +/-2 of the above pH range. Such buffering agent may include Tris (Tris(hydroxymethyl)aminomethane), HEPES (N-[2-hydroxyethyl]-piperazine-N'-[2-ethanesulphonic acid]), PBS (phosphate buffered saline).
The concentration of the buffering agent may be appropriately adjusted according to the buffering agent to be used and the desired pH range.

The present reagent preferably has an osmotic pressure of 150 to 500 mOsm, and more preferably 200 to 350 mOsm. When the reagent has such osmotic pressure, the morphology of cell nuclei can be maintained, so that the fluorescence from the cell nuclei stained with the reagent can be stabilized.

In order to adjust the osmotic pressure of the reagent within the above range, the reagent may comprise an appropriate osmotic pressure adjusting agent. The osmotic pressure adjusting agent may include saccharides, amino acids and sodium chloride. The osmotic pressure can be adjusted by adjusting the concentration of the above buffering agent.
Saccharides may include, but not limited to, monosaccharides (e.g. glucose, fructose), polysaccharides (e.g. arabinose), sugar alcohols (e.g. xylitol, sorbitol, mannitol, ribitol). One or more of these saccharides may be used.
The amino acids may include valine, proline, glycine and alanine.

The concentration of the osmotic pressure adjusting agent may be appropriately decided according to the osmotic pressure adjusting agent to be used. For example, it is preferably about 0.01 to 10 g/L when sodium chloride is used.

The reagent may be mixed with a biological sample containing cervical cells to detect abnormal cells in the sample. The biological sample containing cervical cells may include a swab obtained by smearing of the uterine cervix, tissue obtained from the uterine cervix, a sample solution obtained by an appropriate treatment of them. "Appropriate treatment" includes dipping the swab in an appropriate treating solution such as a buffer preferably comprising a surfactant and suspending cells attached to the swab to the treating solution, or suspending the cervical tissue in an appropriate treating solution such as a buffer preferably comprising a surfactant and optionally homogenizing the suspension.

The above reagent may optionally comprise, in addition to the above component(s), other components such as surfactants, chelating agents, RNase, preservatives and the like.

The surfactant may preferably be a nonionic surfactant. The nonionic surfactant is preferably a polyoxyethylene nonionic surfactant and may include polyoxyethylene (15) oleyl ether, polyoxyethylene (20) oleyl ether, polyoxyethylene (20) stearyl ether, polyoxyethylene (15) cetyl ether and polyoxyethylene (20) cetyl ether. One or more of these surfactants may be used.
The surfactant is preferably contained in the reagent in such concentration that its concentration of 0.01% to 2% is achieved when the reagent is mixed with a biological sample.

The chelating agent may include ethylenediaminetetraacetic acid (EDTA) potassium salt and EDTA sodium salt.
The chelating agent is preferably contained in the reagent in such concentration that its concentration of 1 mM to 200 mM is achieved when the reagent is mixed with a biological sample.

RNase may be incorporated in the present reagent in order to degrade RNA which may be present in a biological sample. RNase may be the one which is commercially available and the one derived from bovine may be mainly used.
RNase is preferably contained in the reagent in such concentration that its concentration of 1 unit to 20 units is achieved when the reagent is mixed with a biological sample.

The preservative is an additive which prevents propagation of microorganisms in the reagent. Specific preservative may include sodium 2-pyridylthio-1-oxide and β-phenethyl alcohol.

The present reagent can be obtained by dissolving the dye of the formula (I) and, if necessary, the optional components into an appropriate solvent. The appropriate solvent is not specifically limited so long as it can dissolve the above component(s). The solvent may be water, methanol, ethylene glycol, dimethylsulphoxide (DMSO) or mixture thereof.
The reagent may be in the solid form which is obtained by dissolving the dye of the formula (I) and the optional component(s) in the appropriate solvent before freeze drying or spray drying the solution. The solid form reagent may be used by dissolving it in the appropriate solvent before use.

The present reagent comprising the dye of the formula (I) may be provided as a single reagent or a reagent kit consisting of a first reagent comprising at least the dye of the formula (I) and a second (or more) reagent(s) comprising any of the above optional component(s).
The reagent kit may include, for example, a reagent kit consisting of a first reagent comprising the dye of the formula (I) and a second reagent comprising the buffering agent; a reagent kit consisting of a first reagent comprising the dye of the formula (I) and the intercalating agent, a second reagent comprising the buffering agent and a third reagent comprising RNase; a reagent kit consisting of a first reagent comprising the dye of the formula (I), a second reagent comprising the intercalating agent, a third reagent comprising the buffering agent and a fourth reagent comprising RNase.

The method of detecting abnormal cells in a biological sample by using the present reagent is also contemplated in the present invention.
The above method comprises the steps of:
preparing a measurement sample by bringing a biological sample containing cells obtained from the uterine cervix into contact with the above cervical abnormal cell detecting reagent;
allowing the measurement sample to flow through a flow cell, irradiating light to the measurement sample in the flow cell and detecting fluorescence emitted from the measurement sample;
obtaining information relating to cell nuclei based on a fluorescent signal generated from the fluorescence detected; and
detecting the abnormal cells in the biological sample based on the obtained information relating to nuclei.
As used herein, "detecting abnormal cells in a biological sample" means detecting the presence or absence of abnormal cells in the biological sample, counting the number of abnormal cells and/or distinguishing abnormal cells from normal cells.

In the above method, a biological sample and the reagent are preferably mixed, when the biological sample is liquid, in the ratio (volume ratio) of biological sample : reagent = 1 : 1 to 50, and more preferably 1 : 8 to 15. When the reagent is in the form of the reagent kit, the biological sample and the total volume of all reagents contained in the reagent kit may be mixed in the above ratio.
When a biological sample is not liquid such as cell pellet, the reagent may be added in such amount that all component(s) in the reagent can achieve the preferable range(s) as described above.

The biological sample and the reagent may be contacted at a temperature of 20°C to 40°C for 0.5 to 20 minutes. The reaction time can be shortened when the reaction temperature is high and it can be extended when the reaction temperature is low.
The biological sample and reagent can be brought into contact by mixing them and leaving them for a determined time period.

The measurement sample prepared by contacting a biological sample with the reagent is then subjected to a flow cell of a flow cytometer.
The flow cytometer to be used may be the conventional flow cytometer comprising a flow cell, a light source, a detecting section which detects emitted light, an analyzing section which analyzes the data derived from the detecting section. The light source of the flow cytometer may be the one having an appropriate wavelength for excitation of the above dye. The light source may be red semiconductor laser, blue semiconductor laser, argon laser, He-Ne laser etc.

In the flow cytometer, the measurement sample is irradiated while flowing through the flow cell. Light to be irradiated has an appropriate wavelength for excitation of the dye, and preferably has the wavelength of 400 to 600 nm.
The fluorescence emitted from the measurement sample to which light having the above wavelength has been irradiated can be detected at the detecting section of the flow cytometer. The fluorescence is not specifically limited and may include side fluorescence and forward fluorescence. The wavelength of the detected fluorescence is preferably 500 to 700 nm.

In the above method, scattered light emitted from the irradiated measurement sample may be also detected. The scattered light is not specifically limited and may include forward scattered light (e.g. receiving angle of around 0 to 20 degrees), side scattered light (receiving angle of abound 90 degrees). Generally, it is known that forward scattered light reflects information on the size of cells and side scattered light reflects internal information of cells such as of nucleus or granules. The width (also referred to as "scattered light pulse width") and intensity (also referred to as "scattered light peak value") of scattered light can be obtained as the scattered light information.

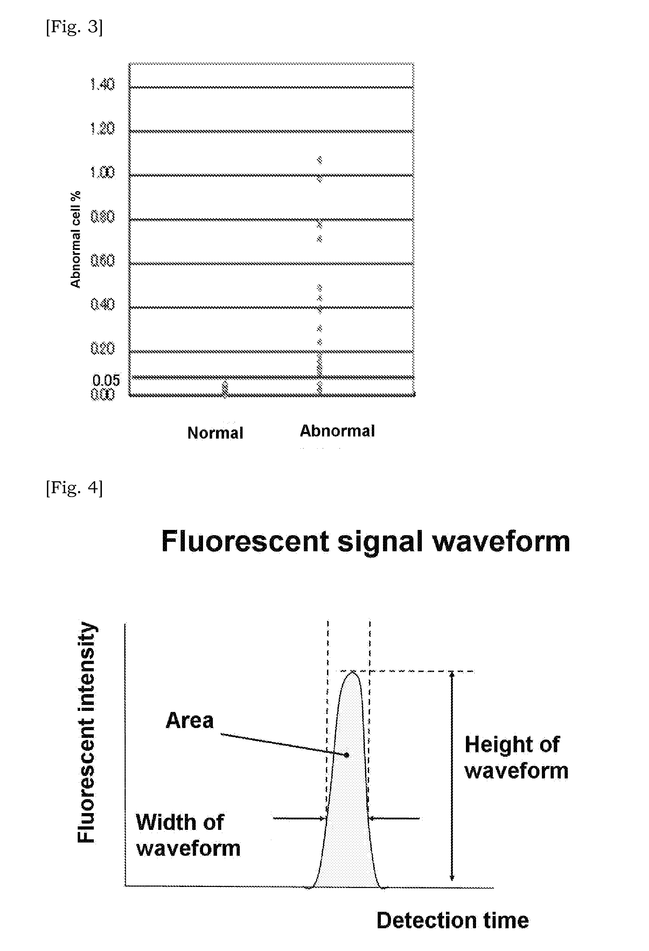

Fluorescence detected at the detecting section may be produced as a fluorescent signal to the analyzing section of the flow cytometer. Based on a waveform of the thus obtained fluorescent signal, information relating to cell nuclei is obtained. The signal waveform of the fluorescent signal can be obtained generally by applying the detected fluorescent intensity to the y-axis and the detection time of the fluorescent signal to the x-axis. An example of such fluorescent signal waveform is shown in Fig. 4.

The information relating to nuclei is preferably at least two selected from information relating to the DNA amount of cells, information relating to chromatin condensation of cells and information relating to nucleolar size of cells. It is more preferable to obtain these three kinds of information.
The information relating to the DNA amount of cells can be provided by the value corresponding to the area of the waveform of the fluorescent signal. The information relating to chromatin condensation of cells can be provided by the value corresponding to the height of the waveform (also referred to as "peak intensity"). The information relating to the nucleolar size of cells can be provided by the value corresponding to the width of the waveform of the fluorescent signal (also referred to as "pulse width").
The analysis of the waveform of the fluorescent signal in order to obtain these values can be performed according to methods which are known in the art per se.

Based on the thus obtained nuclear information, abnormal cells in a biological sample can be detected if the sample contains such cells.
Preferably, the abnormal cells are detected by plotting on a graph the above two or three values capable of providing the nuclear information and carrying out two- or three-dimensional analysis. An appropriate threshold value can be determined by comparing the values obtained by the analyses on biological samples containing normal cells and abnormal cells, which are judged by a cytological diagnosis method that is known per se. Such analysis methods are known to a skilled person. Cells presenting the value on or above (or below) such threshold value can be judged as abnormal cells. According to such method, the presence or absence of abnormal cells in a biological sample can be detected.

The dye of the formula (I) contained in the reagent used in the present method is believed to bind to a minor groove of DNA in cell nuclei, as mentioned above. Thus, fluorescence emitted from the dye is believed to be able to provide information relating to cell nuclei. As described, it is known that abnormal cells have such abnormalities as change in the size of nuclei, increase in the DNA amount or chromatin condensation; therefore, information based on fluorescence from abnormal cells is considered to be different from that from normal cells. The present method makes it possible to distinguish abnormal cells from normal cells by utilizing such difference.

The number of the detected abnormal cells can also be counted based on the above nuclear information. The ratio of the number of abnormal cells counted relative to the number of the total epithelial cells may be calculated, and, based on such ratio, a biological sample containing abnormal cells can be distinguished from the one containing normal cells.
The number of the total epithelial cells can be obtained based on the scattered light which can be measured as described above.

### Examples

The present invention is further illustrated by the following Examples, which do not limit the present invention.

### Preparation Examples of Dyes

The dyes which can be used in the present invention were prepared as follows.

### Preparation Example 1

Dye A was prepared as follows.

In a reaction vessel, 7.6 g of a compound (2-mercaptobenzothiazole) represented by the following formula (1): 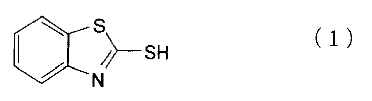 and 6.5 g of a compound (4-methylquinoline) represented by the following formula (2): 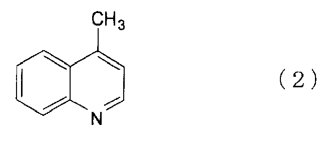 were reacted while heating and stirring for 5 hours in diethyl sulphate. Acetonitrile and triethylamine were added and the mixture was heated for two hours to allow reaction. The reaction mixture was cooled down and deposited crystals were recovered and reacted with 6.8 g of sodium iodide in methanol under heating for one hour. The reaction mixture was cooled down and deposited crystals were filtered out. The red crystals of a mono-methine compound (Dye A) were obtained (8.4 g). λmax 503 nm, m.p. 304°C
¹H-NMR δ (TMS, ppm) 1.37-1.51 (6H, dt, CH3), 4.67 (4H, m, CH2), 6.93 (1H, s, CH), 7.39-7.42 (2H, m, ArH), 7.62 (1H, t, ArH), 7.75-7.80 (2H, m, ArH), 7.98-8.17 (3H,m, ArH), 8.66-8.81 (2H, m, ArH).

### Preparation Example 2

Dye B was prepared as follows.
The compound represented by the formula (1) and the compound represented by the formula (2) were reacted in dimethyl sulphate in the similar manner as Preparation Example 1 to give dark red crystals of a mono-methine compound (Dye B).
λmax 502 nm, m.p. 288°C
¹H-NMR δ (TMS, ppm) 3.98 (3H, s, CH3), 4.15 (3H, s, CH3), 6.87 (1H, s, CH), 7.29 (1H, dd, ArH), 7.39 (1H, t, ArH), 7.58 (1H, t, ArH), 7.71-7.78 (2H, m, ArH), 7.96-8.02 (3H, m, ArH), 8.59 (1H, d, ArH), 8.78 (1H, d, ArH).

### Preparation Example 3

Dye C was prepared as follows.
The reaction was carried out in dimethyl sulphate in the similar manner as Preparation Example 1 except that the compound represented by the following formula (4): 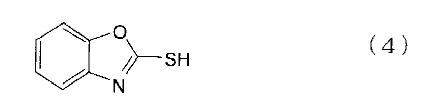 was used instead of the compound represented by the formula (1), to give orange coloured crystals of a mono-methine compound (Dye C). λmax 476 nm, m.p. 298°C
¹H-NMR δ (TMS, ppm) 3.86 (3H, s, CH3), 4.16 (3H, s, CH3), 6.27 (1H, s, CH), 7.38 (1H, t, ArH), 7.48 (1H, t, ArH), 7.64 (1H, d, ArH), 7.72-7.81 (2H, m, ArH), 7.92-8.05 (3H, m, ArH), 8.46 (1H, d, ArH), 8.79 (1H, d, ArH).

### Preparation Example 4

Dye D was prepared as follows.
The reaction was carried out in dimethyl sulphate in the similar manner as Preparation Example 1 except that the compound represented by the following formula (7): 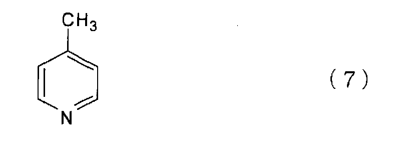 was used instead of the compound represented by the formula (2), to give yellow crystals of a mono-methine compound (Dye D).
λmax 445 nm, m.p. 299°C
¹H-NMR δ (TMS, ppm) 3.72 (3H, s, CH3), 3.98 (3H, s, CH3), 6.25 (1H, s, CH), 7.30 (1H, t, ArH), 7.41 (2H, d, ArH), 7.49 (1H, t, ArH), 7.58 (1H, d, ArH), 7.90 (1H, d, ArH), 8.28 (2H, d, ArH).

### Preparation Example 5

Dye E was prepared as follows.
The reaction was carried out in dimethyl sulphate in the similar manner as Preparation Example 1 except that the compound represented by the following formula (3): 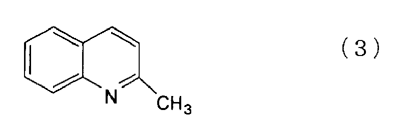 was used instead of the compound represented by the formula (2), to give red-orange crystals of a mono-methine compound (Dye E).
λmax 436 nm, m.p. 305°C
¹H-NMR δ (TMS, ppm) 3.78 (3H, s, CH3), 4.07 (3H, s, CH3), 5.83 (1H, s, CH), 7.22-7.34 (2H, m, ArH), 7.52 (1H, t, ArH), 7.61 (1H, d, ArH), 7.89 (2H, dd, ArH), 8.16 (1H, t, ArH), 8.53 (1H, d, ArH).

### Production Example 6

Dye F was prepared as follows.
The reaction was carried out in dimethyl sulphate in the similar manner as Preparation Example 1 except that the compound represented by the following formula (8): 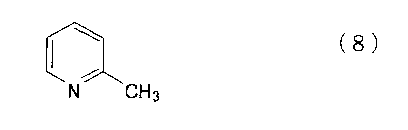 was used instead of the compound represented by the formula (2), to give yellow-orange crystals of a mono-methine compound (Dye F). λmax 482 nm, m.p. 280°C
¹H-NMR δ (TMS, ppm) 3.95 (3H, s, CH₃), 4.13 (3H, s, CH₃), 6.16 (1H, s, CH), 7.42 (1H, t, ArH), 7.58 (2H, t, ArH), 7.79 (2H, d, ArH), 7.90 (1H, t, ArH), 7.93-8.15 (4H, m, ArH), 8.46 (1H, d, ArH).

### Production Example 7

Dye G was prepared as follows.
The compound represented by the formula (1) and the compound represented by the formula (2) were reacted in ethyl p-toluenesulphonate in the similar manner as Preparation Example 1 to give red-orange crystals of a mono-methine compound (Dye G).
λmax 503 nm, m.p. 221°C
¹H-NMR δ (TMS, ppm) 1.39 (3H, t, CH3), 1.48 (3H, t, CH3), 2.28 (3H, s, CH3), 4.66 (4H, m, CH2), 6.93 (1H, s, CH), 7.11 (2H, d, ArH), 7.34-7.50 (4H, m, ArH), 7.63 (1H, t, ArH), 7.76 (2H, m, ArH), 7.97-8.05 (2H, m, ArH), 8.16 (1H, d, ArH), 8.66 (1H, d, ArH), 8.80 (1H, d, ArH).

### Preparation Example 8

Dye H was prepared as follows.
The compound represented by the formula (1) and the compound represented by the formula (2) were reacted in propane sultone in the similar manner as Preparation Example 1 to give red-orange crystals of a mono-methine compound (Dye H).
λmax 504 nm, m.p. 303°C
¹H-NMR δ (TMS, ppm) 1.17 (9H, t, CH3), 2.14-2.21 (4H, m, CH2), 2.54 (2H, t, CH2), 2.71 (2H, t, CH2), 3.05-3.14 (6H, m, CH2), 4.73-4.82 (4H, m, CH2), 7.16 (1H, s, CH), 7.39 (2H, m, ArH), 7.61 (1H, t, ArH), 7.68 (1H, t, ArH), 7.85 (1H, d, ArH), 7.87-8.02 (2H, m, ArH), 8.23 (1H, d, ArH), 8.64 (1H, d, ArH), 8.88 (1H, br, OH), 9.08 (1H, d, ArH).

### Preparation Example 9

Dye J was prepared as follows.
The reaction was carried out in dimethyl sulphate in the similar manner as Preparation Example 1 except that the compound represented by the following formula (5): 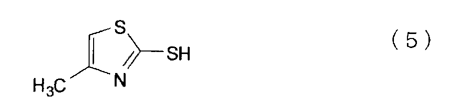 was used instead of the compound represented by the formula (1), to give bright red crystals of a mono-methine compound (Dye J).
λmax 507 nm, m.p. 340°C
¹H-NMR δ (TMS, ppm) 2.41 (3H, s, CH3), 3.82 (3H, s, CH3), 4.00 (3H, s, CH3), 6.73 (1H, s, CH), 6.91 (1H, d, ArH), 7.20 (1H, s, ArH), 7.61-7.66 (1H, br, ArH), 7.87-7.92 (2H, m, ArH), 8.26 (1H, d, ArH), 8.65 (1H, d, ArH).

### Preparation Example 10

Dye K was prepared as follows.
The reaction was carried out in dimethyl sulphate in the similar manner as Preparation Example 1 except that the compound represented by the following formula (6): 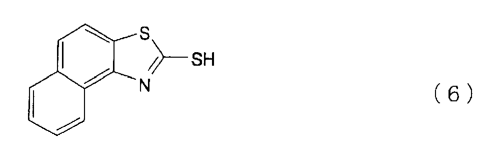 was used instead of the compound represented by the formula (1), to give brown crystals of a mono-methine compound (Dye K).
λmax 523 nm, m.p. 253°C
¹H-NMR δ (TMS, ppm) 4.04 (3H, s, CH3), 4.38 (3H, s, CH3), 6.87 (1H, s, CH), 7.15 (1H, d, ArH), 7.61-7.71 (3H, m, ArH), 7.83-8.04 (5H, m, ArH), 8.45 (1H, d, ArH), 8.62 (1H, d, ArH), 8.70 (1H, d, ArH).

### Preparation Example 11

Dye L was prepared as follows.
The reaction was carried out in the similar manner as Preparation Example 1 except that the compound represented by the formula (4) was used instead of the compound represented by the formula (1) to give bright red crystals of a mono-methine compound (Dye L). λmax 477 nm, m.p. 276°C
¹H-NMR δ (TMS, ppm) 1.39 (3H, t, CH3), 1.47 (3H, t, CH3), 4.48 (2H, q, CH2), 4.64 (2H, q, CH2), 6.32 (1H, s, CH), 7.39 (1H, t, ArH), 7.48 (1H, t, ArH), 7.66-7.82 (3H, m, ArH), 7.96-8.02 (2H, m, ArH), 8.14 (1H, d, ArH), 8.53 (1H, d, ArH), 8.82 (1H, d, ArH).

The solutions were prepared as follows.

### (1) Dilution solution

The following components:
Tris (Wako Pure Chemical Industries: 207-06275) 2.422g (20 mM) Sodium chloride (Wako Pure Chemical Industries: 191-01665) 5.1427g EDTA-2K (Dojindo Laboratories: K001) 16.178g (40 mM) Polyoxyethylene (20) oleyl ether (BO-20: Nikko Chemicals) 0.5g (0.05%) were weighed and dissolved in about 800 ml of milli-Q water. pH was adjusted to 8.0 + 0.05 with NaOH, the volume was adjusted to 1000 ml with water and the solution was filtered with a 0.22-µl filter. The obtained dilution solution had an osmotic pressure of 300 mOsm.

### (2) Dye solution

### (2-1) Propidium iodide (PI) solution

PI powder (Sigma, P4170, lot 037K3676; 100 mg) as the intercalating dye was weighed and dissolved in 50 ml of methanol. The volume was adjusted to 500 ml with milli-Q water to obtain the PI solution.

### (2-2) Dye A solution

About 100 mg of Dye A prepared as Preparation Example 1 was weighed and dissolved in 50 ml of dimethylsulphoxide (DMSO). A 10-ml portion was taken and milli-Q water was added to 1000 ml to obtain the Dye A solution.

### (2-3) PI + Dye A solution

The equivalent volumes of above prepared PI solution and Dye A solution were mixed to obtain PI + Dye A solution.

### (3) RNase solution

RNase (Sigma: R4642) was diluted to 6.6 units with Tris HCl (pH 8.0) solution.

### Examples 1 and 2 and Comparative Example 1

The samples (n=38) were obtained by using swabs from the uterine cervices of subjects and suspending them in a buffer. Among them, 33 samples contained normal cells and 5 samples contained abnormal cells, as verified by the conventional cytological diagnosis method, Papanicolaou staining.
The number of cells in samples was counted, and the samples containing 10⁵ cells were transferred to tubes and centrifuged at 10,000 rpm for 1 minute at room temperature. The supernatant was removed with an aspirator and 1 mL of the dilution solution prepared in the above (1) was added. Cells were suspended with pipetting for five times and the suspension was centrifuged at 10,000 rpm for 1 minute at room temperature. The supernatant was removed with an aspirator to wash cells.

The following solutions were added to the above cells (biological samples) (the dilution solution was heated at 37°C) and mixed by pipetting, and the mixture was left to stand in the dark at 37°C for 5 minutes to stain the cells.

**[Table 1]**

| | Comp. Ex. 1 | Ex. 1 | Ex. 2 |
|---|---|---|---|
| Dilution solution (µl) | 330 | 330 | 310 |
| Dye solution (µl) | 20 | 20 | 40 |
| RNase solution (µl) | 20 | 20 | 20 |
| Total (µl) | 370 | 370 | 370 |

The Dye solutions used were PI solution, Dye A solution and PI + Dye A solution for Comparative Example 1, Example 1 and Example 2, respectively. The concentrations of the dyes in the mixture with the biological sample were 10 µg/mL for Comparative Example 1, 1 µg/mL for Example 1 and 10 µg/mL and 1 µg/mL for PI and Dye A in Example 2.

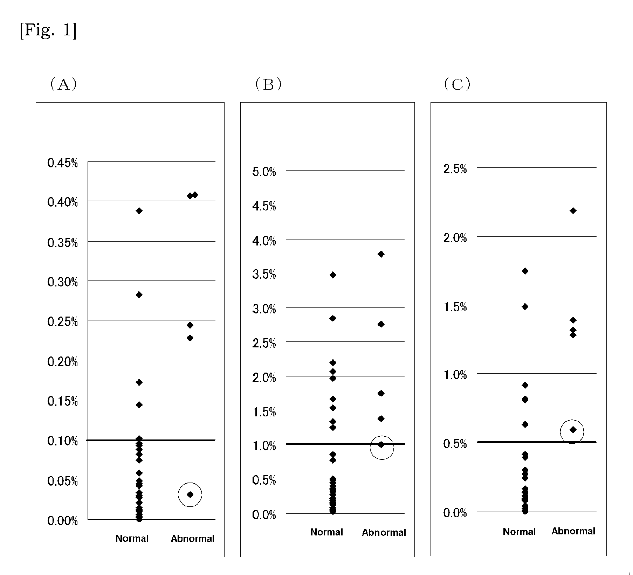

The obtained measurement samples were subjected to a flow cytometer that comprises the light source of a semiconductor laser irradiating light having the wavelength of 473 nm, and forward scattered light and fluorescence were measured to obtain forward scattered light pulse width, forward scattered light peak value, fluorescence peak value and fluorescence pulse value. First, a scattergram was generated based on the forward scattered light pulse width and forward scattered light peak value to differentiate and count the cervical epithelial cells apart from other cells ("number of epithelial cells"). Then, a scattergram was prepared based on the fluorescence peak value and fluorescence pulse width, and the cells appearing in the region on or above a determined threshold value were considered as abnormal cells, whose number was then counted ("number of abnormal cells"). The ratio of abnormal cells was calculated by dividing the number of abnormal cells by the number of epithelial cells. The results are shown in Fig. 1.

Fig. 1 shows the results of (A) Comparative Example 1, (B) Example 1 and (C) Example 2. "Normal" represents the results of the biological samples as judged to contain normal cells by Papanicolaou staining, and "Abnormal" represents the results of the biological samples as judged to contain abnormal cells by Papanicolaou staining.

According to the results of Comparative Example 1 in which the conventional PI was used, one sample which contains abnormal cells may be judged to be "Normal" when the threshold value for distinguishing the biological samples containing normal cells and abnormal cells is determined to be "0.10%". However, when this threshold value is lowered in order to judge this abnormal sample as "Abnormal", most of the biological samples which contain normal cells may erroneously be judged as "Abnormal".
On the other hand, in Examples 1 and 2 in which the present reagents are used, all biological samples containing abnormal cells can be judged to contain abnormal cells, and most of biological samples containing normal cells can be judged as normal.

### Example 3

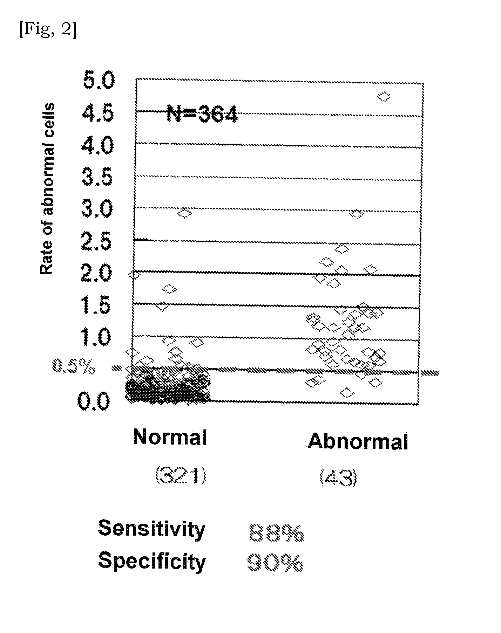

In the similar manner as Example 2, 364 samples were measured. Among these samples, 321 samples contained normal cells and 43 samples contained abnormal cells, as judged by the cytological diagnosis (Papanicolaou staining).
The results are shown in Fig. 2 and Table 2.

**[Table 2]**

| | Cytological diagnosis (+) | Cytological diagnosis (-) |
|---|---|---|
| Present method (+) | 38 | 33 |
| Present method (-) | 5 | 288 |

The results shown in Fig. 2 and Table 2 show that among 321 samples which were judged as normal by cytological diagnosis, 288 samples and 33 samples could be judged as normal and abnormal samples, respectively, when the threshold value for distinguishing the biological samples containing abnormal cells and normal cells is determined to be 0.5%. In addition, it is also found that among 43 samples which were judged as abnormal by cytological diagnosis, 38 samples and 5 samples could be judged as normal and abnormal samples, respectively.
Thus, the "sensitivity" of the method in which the present reagent was used, which corresponds to the ratio for judging abnormal samples as "abnormal", was 88% (= 38/43) and the "specificity" of the method, which corresponds to the ratio for judging normal samples as "normal", was 90% (= 288/321).

### Example 4

The solutions were prepared as follows.

### (1) Dilution solution

The following components:
Tris (Wako Pure Chemical Industries: 207-06275) 2.422g (20 mM) EDTA-2K (Dojindo Laboratories: K001) 16.178g (40 mM) Polyoxyethylene (20) oleyl ether (BO-20: Nikko Chemicals) 0.5g (0.05%) were weighed and dissolved in about 800 ml of milli-Q water. pH was adjusted to 7.2 + 0.05 with NaOH, the volume was adjusted to 1000 ml with water and the solution was filtered with a 0.22-µl filter. The obtained dilution solution had an osmotic pressure of 230 mOsm.

### (2) Dye solution (PI + Dye A solution)

PI powder (200 mg) was weighed and dissolved in 50 ml of methanol. The volume was adjusted to 500 ml with milli-Q water to obtain the PI solution. About 100 mg of Dye A was weighed and dissolved in 50 ml of DMSO. A 10-ml portion was taken and milli-Q water was added to 1000 ml to obtain a Dye A solution. The equivalent volumes of these two solutions were mixed to obtain PI + Dye A solution.

### (3) RNase solution

It was prepared as described in Example 2.

In the present Example, 137 samples were used. Among them, 113 samples contained normal cells and 23 samples contained abnormal cells, as verified by the cytological diagnosis (Papanicolaou staining).
To the cells (biological samples) washed according to the procedure described in Example 2 were added 330 µl of the dilution solution heated at 37°C, 20 µl of the RNase solution and 20 µl of the dye solution and mixed by pipetting, and the mixture was left to stand in the dark at 37°C for 5 minutes to stain the cells. The concentrations of the dyes in the mixtures with the biological samples were 10 µg/mL and 0.5 µg/mL for PI and Dye A, respectively.

The obtained measurement samples were subjected to a flow cytometer that comprises the light source of a semiconductor laser irradiating light having the wavelength of 473 nm, and forward scattered light and fluorescence were measured to obtain forward scattered light pulse width, forward scattered light peak value, fluorescence peak area value, fluorescence peak value and fluorescence pulse value. A scattergram was generated based on the forward scattered light pulse width and forward scattered light peak value to differentiate and count the cervical epithelial cells apart from other cells ("number of epithelial cells"). Then, a three-dimensional scattergram was prepared based on the fluorescence peak area value, the fluorescence peak value and the fluorescence pulse width, and the cells appearing in the region on or above a determined threshold value were considered as abnormal cells, whose number was then counted ("number of abnormal cells"). The ratio of abnormal cells was calculated by dividing the number of abnormal cells by the number of epithelial cells. The obtained results are shown in Fig. 3 and Table 3.

**[Table 3]**

| | Cytological diagnosis (+) | Cytological diagnosis (-) |
|---|---|---|
| Present method (+) | 21 | 4 |
| Present method (-) | 2 | 109 |

The results shown in Fig. 3 and Table 3 show that among 113 samples which were judged as normal by cytological diagnosis, 109 samples and 4 samples could be judged as normal and abnormal samples, respectively, when the threshold value for distinguishing the biological samples containing abnormal cells and normal cells is determined to be 0.05%. In addition, it was found that among 23 samples which were judged as abnormal by cytological diagnosis, 21 samples and 2 samples could be judged as normal and abnormal samples, respectively.
Thus, the "sensitivity" of the method in which the present reagent was used, which corresponds to the ratio for judging abnormal samples as "abnormal", was 91% (= 21/23) and the "specificity" of the method, which corresponds to the ratio for judging normal samples as "normal", was 96% (= 109/113).

It is found that by using the present reagent, abnormal cells in biological samples obtained from the uterine cervix can be detected more accurately as well as rapidly and conveniently.

### Example 5

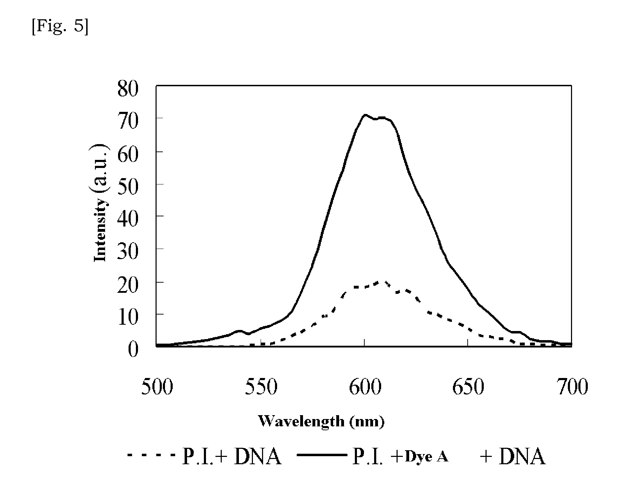

Fig. 5 shows the fluorescent intensities of salmon sperm DNA (0.09 mg/ml) in Tris-borate buffer (TBS: pH 8.0) which was stained with:
(1) PI (25 µg/ml); or
(2) PI (25 µg/ml) and Dye A (1 µg/ml).

The results of Fig. 5 show that the fluorescent signal is increased when DNA is stained with PI and Dye A compared to the one observed with the staining with PI. This demonstrates that Dye A intensifies fluorescence of PI, suggesting that the reagent containing both PI and Dye A allows more sensitive detection of abnormal cells in biological samples obtained from the uterine cervix.

### Example 6

The dilution solution, staining solution and RNase solution of Example 4 were used to prepare measurement samples from 211 samples. Among these 211 samples, 185 samples did not contain abnormal cells and 26 samples contained abnormal cells, as verified by the cytological diagnosis (Papanicolaou staining).

The obtained measurement samples were subjected to a flow cytometer that comprises the light source of a semiconductor laser irradiating light having the wavelength of 473 nm, and forward scattered light and fluorescence were measured to obtain fluorescence peak area value, fluorescence peak value and fluorescence pulse width.

The measurement samples which have the fluorescence peak area values 2.5-fold or more higher than the modal fluorescence peak area value of 185 samples without abnormal cells were judged as positive (+) and those having the values less than 2.5-fold of the modal fluorescence peak area value of 185 samples were judged as negative (-). The comparison of the results obtained by the judgments based on the fluorescence peak area values only and on the cytological diagnosis is shown in Table 4.

**[Table 4]**

| | | Cytological diagnosis | |
|---|---|---|---|
| | | (+) | (-) |
| Judgment based on fluorescence peak area value | (+) | 22 | 36 |
| | (-) | 4 | 149 |

The results in Table 4 show that the sensitivity and specificity of the judgment based on the fluorescence peak area values only were 85% (22/26) and 81% (149/185), respectively.

Next, the measurement samples which have the fluorescence peak area values and fluorescence peak values 2.5-fold or more higher than the modal fluorescence peak area value and the modal fluorescence peak value, respectively, of 185 samples without abnormal cells were judged as positive (+) and those having the values less than the 2.5-fold values, respectively, were judged as negative (-). The comparison of the results obtained by the judgments based on the fluorescence peak area values in addition to the fluorescence peak values and on the cytological diagnosis is shown in Table 5.

**[Table 5]**

| | | Cytological diagnosis | |
|---|---|---|---|
| | | (+) | (-) |
| Judgment based on fluorescence peak area value and fluorescence peak value | (+) | 23 | 39 |
| | (-) | 3 | 146 |

The results in Table 5 show that the sensitivity and specificity of the judgments based on the fluorescence peak area values and fluorescence peak values were 89% (22/26) and 79% (149/185), respectively.

Next, the measurement samples which have the fluorescence peak area values and fluorescence peak values 2.5-fold or more higher than the modal fluorescence peak area value and the modal fluorescence peak value, respectively, of 185 samples without abnormal cells and which have 60 or more fluorescence pulse width value were judged as positive (+) and other measurement samples were judged as negative (-). The comparison of the results obtained by the judgments based on the fluorescence peak area values, the fluorescence peak values and the fluorescence peak values, and on the cytological diagnosis is shown in Table 6.

**[Table 6]**

| | | Cytological diagnosis | |
|---|---|---|---|
| | | (+) | (-) |
| Judgment based on fluorescence peak area value , fluorescence peak value and fluorescence pulse widths | (+) | 25 | 38 |
| | (-) | 1 | 147 |

The results in Table 5 show that the sensitivity and specificity of the judgments based on the fluorescence peak area values, fluorescence peak values and fluorescence pulse widths were 96% (25/26) and 79% (147/185), respectively.

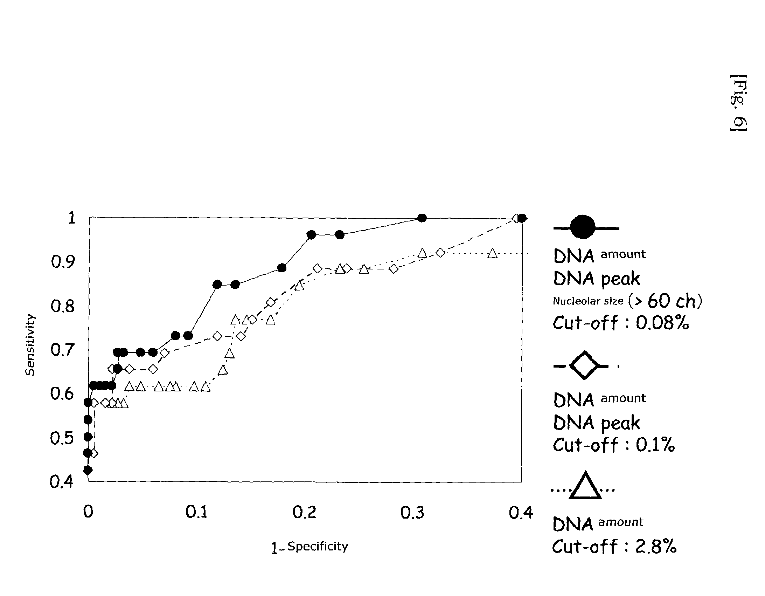

Fig. 6 shows the results of receiver operating characteristic (ROC) analysis of the judgments based on (1) the fluorescence peak area values (Δ), (2) the fluorescence peak area values and the fluorescence peak values (0) and (3) the fluorescence peak area values, the fluorescence peak values and the fluorescence pulse widths (•).

The results in Fig. 6 show that the accuracy of the detection of abnormal cells is improved in the order of the judgments based on (1) the fluorescence peak area values, (2) the fluorescence peak area values and the fluorescence peak values and (3) the fluorescence peak area values, the fluorescence peak values and the fluorescence pulse widths. Accordingly, it is suggested that, when abnormal cells are detected in biological samples obtained from the uterine cervix, the use of multiple parameters rather than one parameter among fluorescence peak area value, fluorescence peak value and fluorescence pulse width allows more sensitive detection of abnormal cells. Particularly, the use of all parameters of fluorescence peak area value, fluorescence peak value and fluorescence pulse width allows further more sensitive detection of abnormal cells.

The present application relates to Japanese Patent Application No. 2008-196372 filed on July 30, 2008, whose claims, specification, drawings and abstract are incorporated herein by reference.

## Claims

1. A cervical abnormal cell detecting reagent for detecting abnormal cells in a biological sample containing cells obtained from the uterine cervix, **characterized in that** it comprises a compound having the following general formula (I): 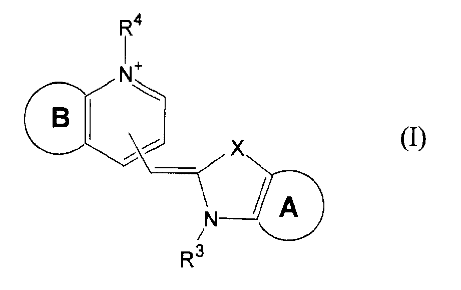 wherein:
X represents S or O; 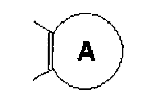 represents 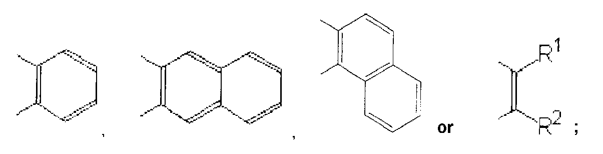 R¹ and R², which are identical or different, represent a hydrogen atom or a C₁₋₆ alkyl group; 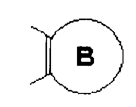 represents 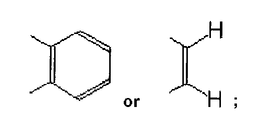 and
R³ and R⁴, which are identical or different, represent a C₁₋₆ alkyl group which may have a sulphonic acid group as a substituent,
or a salt thereof.

2. The cervical abnormal cell detecting reagent according to claim 1, which further comprises an intercalating dye selected from propidium iodide, ethidium bromide and acridine orange.

3. The cervical abnormal cell detecting reagent according to claim 1, which has pH of 7 to 8.5.

4. The cervical abnormal cell detecting reagent according to claim 1, which has an osmotic pressure of 200 to 350 mOsm.

5. The cervical abnormal cell detecting reagent according to claim 1, wherein the compound of the formula (I) is the salt and a counter ion is an anion.

6. The cervical abnormal cell detecting reagent according to claim 1, wherein the abnormal cells are cancer cells or atypical cells.

7. A method of detecting abnormal cells in a biological sample containing cells obtained from the uterine cervix comprising the steps of:
preparing a measurement sample by bringing the biological sample into contact with the cervical abnormal cell detecting reagent according to claim 1;
allowing the measurement sample to flow through a flow cell, irradiating light to the measurement sample in the flow cell and detecting fluorescence emitted from the measurement sample;
obtaining information relating to cell nuclei based on a fluorescent signal generated from the fluorescence detected; and
detecting the abnormal cells in the biological sample based on the obtained information relating to nuclei.

8. The method according to claim 7, wherein the information relating to cell nuclei is at least two selected from information relating to DNA amount of cells, information relating to chromatin condensation of cells and information relating to nucleolar size of cells.

9. The method according to claim 8, wherein the information relating to DNA amount of cells is a value corresponding to an area of a waveform of the fluorescent signal; the information relating to chromatin condensation of cells is a value corresponding to a height of a waveform of the fluorescent signal; and the information relating to nucleolar size of cells is a value corresponding to a width of a waveform of the fluorescent signal.

10. The method according to claim 7, wherein the abnormal cells are cancer cells or atypical cells.
